# EUROPEAN PATENT SPECIFICATION

(11) **EP 4 001 882 B1**
(45) Date of publication and mention of the grant of the patent: **02.07.2025**
(21) Application number: 21208581.5
(22) Date of filing: 16.11.2021
(51) Int. Cl.: G01N 1/22, B01L 7/00, B64D 11/04, B64D 11/00

(54) **SYSTEMS AND METHODS FOR COLLECTING A BIOLOGICAL SAMPLE FROM A PASSENGER CABIN**
SYSTEME UND VERFAHREN ZUR ENTNAHME EINER BIOLOGISCHEN PROBE AUS EINER PASSAGIERKABINE
SYSTÈMES ET PROCÉDÉS DE COLLECTE D'UN ÉCHANTILLON BIOLOGIQUE DANS UNE CABINE DE PASSAGERS

(30) Priority: 16.11.2020 US 202063114339 P; 16.11.2020 US 202063114330 P; 16.11.2020 US 202063114400 P; 16.11.2020 US 202063114064 P; 16.11.2020 US 202063114157 P; 16.11.2020 US 202063114386 P; 16.11.2020 US 202063114366 P; 16.11.2020 US 202063114350 P
(43) Date of publication of application: 25.05.2022
(73) Proprietor: B/E Aerospace, Inc., Winston-Salem, NC 27105 (US)
(72) Inventor: GONZALEZ, Arnau Castillo, 3607-AK Maarseen (NL); GONZALEZ, Vanessa, CP 07004 Palma de Mallorca (ES); ST. ROCK, Brian, Andover, CT 06232 (US); MURCIA MARTINEZ, Antonio, 03206 Elche, Alicante (ES)
(74) Representative: Dehns

(56) References cited:
- US-A1- 2008 299 648
- US-A1- 2012 122 075
- US-A1- 2013 137 183
- US-A1- 2015 136 602
- US-A1- 2016 025 603

## Description

### BACKGROUND OF THE INVENTION

### 1. Field of the Invention

The present disclosure relates to collecting a biological sample and more particularly to systems and methods for collecting a biological sample representative of a passenger cabin on an aircraft using a collector.

### 2. Description of Related Art

The spread progression of SARS-CoV-2 around the world has brought attention to the systemic risks of economic globalization. As a result of the COVID-19 pandemic there is a need for better monitoring, detecting, and isolating ill passengers, specifically due to the detrimental impact on the global economy, to prevent the spread of COVID-19 and other pathogens during travel, e.g. air travel, rail travel or the like, due to closed borders, movement restrictions, and testing requirements.

The COVID-19 pandemic the air travel industry has proven that air travel can be safe and that aircraft cabins have a well-managed airflow that inhibits transmission of virus, and that being seated onboard an aircraft is safer than shopping in large stores. Governments and other authorities, however, need to assume that aircraft are contaminated until proven "clean", as 25% of COVID-19 cases are asymptomatic or pre-symptomatic; but still contagious. To date, travelers and governments have relied on individual tests. Testing for viruses requires that samples be taken of various bodily tissues and/or fluids. An adequate concentration of material is needed, the concentration being determined by the type and sensitivity of the testing procedure. It could be difficult to get adequate samples from travelers for the purposes of virus detection, contact tracing in the event of an exposure, etc. It is also possible that the airline is required to certify the arriving aircraft as being "virus free".

The conventional techniques have been considered satisfactory for their intended purpose. However, there is an ever present need for improved monitoring, detecting, and isolating systems and methods. This disclosure provides a solution for this need.

US 2013/137183 A1 relates to the detection of prohibited goods. US 2016/025603 A1 relates to the fields of microbiology, pathology and air handling. US2012/122075 A1 relates to system and method for detecting threatening agents in the air.

### SUMMARY

A system for collecting a biological sample from a passenger cabin is provided as claimed in claim 1 and includes a collector for collecting particulate samples of the biological sample positioned within at least one of a passenger cabin or a cabin air outflow flow path.

The collector includes a continuous strip of collector material positioned along a lengthwise side of the passenger cabin between a hull wall and the passenger cabin. The collector can include a series of individual pieces of collector material spaced apart along a lengthwise side of the passenger cabin between a hull wall and the passenger cabin. Each individual piece of collector material can be spaced apart by a given number of rows of seats.

The collector can include a plurality of individual pieces of collector material below a row of seats. The plurality of individual pieces of collector material can be more proximate to a hull wall than a cabin aisle. The collector can include a group of individual pieces of collector material on a hull wall or a passenger seat back at a height more proximate to a top of a passenger seat than a cabin floor. The cabin air outflow flow path can be defined by a space between an aircraft hull and a cabin floor. The collector can be positioned within the cabin air outflow flow path. The outflow flow path can be defined at least in part through an in-flight entertainment system (IFE) filter racks. The collector can include a plurality of individual pieces of filter material. The cabin air outflow flow path can be a galley insert air outflow flow path. The collector can be positioned within the cabin air outflow flow path. The cabin air outflow flow path can be a cabin temperature outflow flow path.

The particulate samples can include droplets exhaled from passengers throughout a duration of a flight. The collector can be configured and adapted to be removed from the passenger cabin or cabin air outflow flow path for testing. The cabin air outflow flow path can be a general cabin outlet flow path and wherein the collector can be positioned across the general cabin outlet flow path. The system can include an aircraft galley proximate to the passenger cabin and a pathogen identifying testing unit within the aircraft galley. The pathogen identifying testing unit can include a communication unit for communicating results remotely. In certain embodiments, the pathogen identifying testing unit can include a Polymerase Chain Reaction (PCR) testing unit.

In accordance with another aspect, a method for collecting particulates from cabin air is provided as claimed in claim 13 includes capturing particulates of a biological sample in at least one of a passenger cabin or a cabin air outflow flow path with a collector for a period of time. The method includes removing the collector from at least one of the passenger cabin or air outflow flow path for testing, the collector including a continuous strip of collector material positioned along a lengthwise side of the passenger cabin between a hull wall and the passenger cabin. The method includes placing a clean collector into at least one of the passenger cabin or a cabin air outflow flow path for use during another period of time.

The method can include conducting a pathogen identifying test on at least one particulate captured in the collector. The method includes relaying a result of the pathogen identifying test to a central data center. In certain embodiments, conducting the pathogen identifying test can include conducting a PCR test on at least one particulate captured in the collector.

These and other features of the systems and methods of the subject disclosure will become more readily apparent to those skilled in the art from the following detailed description of the preferred embodiments taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, preferred embodiments thereof will be described in detail herein below with reference to certain figures, wherein:
Fig. 1 is a schematic cross-sectional view of an embodiment of a system for collecting a biological sample from a passenger cabin constructed in accordance with the present disclosure, showing a collector positioned within the passenger cabin in a cabin air outflow flow path;
Fig. 2 is a schematic top plan view of the system of Fig. 1, showing a continuous strip of collector material positioned along lengthwise sides of the passenger cabin;
Fig. 3A is a schematic top plan view of another embodiment of a system for collecting a biological sample from a passenger cabin constructed in accordance with the present disclosure, showing separate strips of collector material spaced apart along lengthwise sides of the passenger cabin;
Fig. 3B is a schematic top plan view of the system of Fig. 3A, showing a separate strip of collector material positioned along a lengthwise side of the passenger cabin;
Fig. 4 is a perspective view of a portion of the system of Fig. 1, showing a collector positioned in a galley insert air outflow flow path; and
Fig. 5 is a schematic top plan view of a portion of the system of Fig. 1, showing a pathogen identifying testing unit for communicating results remotely.

### DETAILED DESCRIPTION OF THE PREFERRED EMBODIMENTS

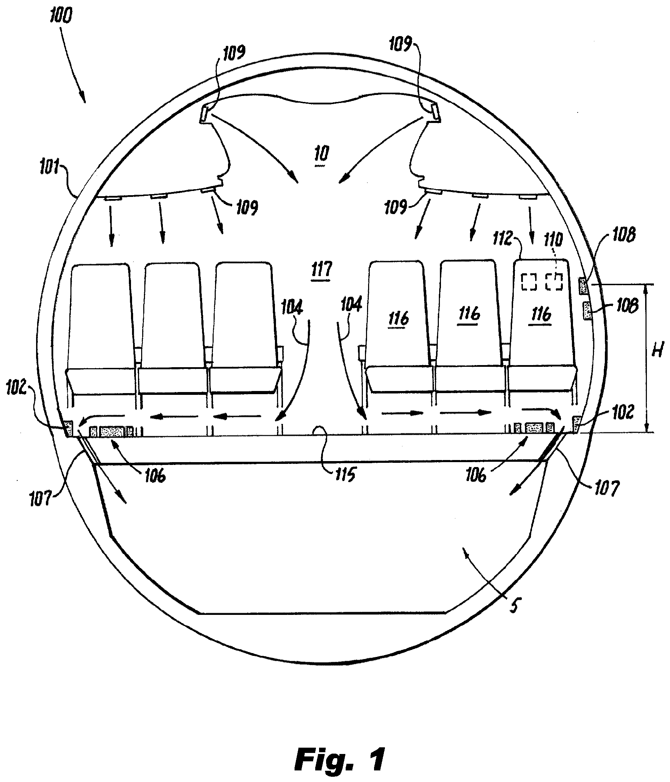

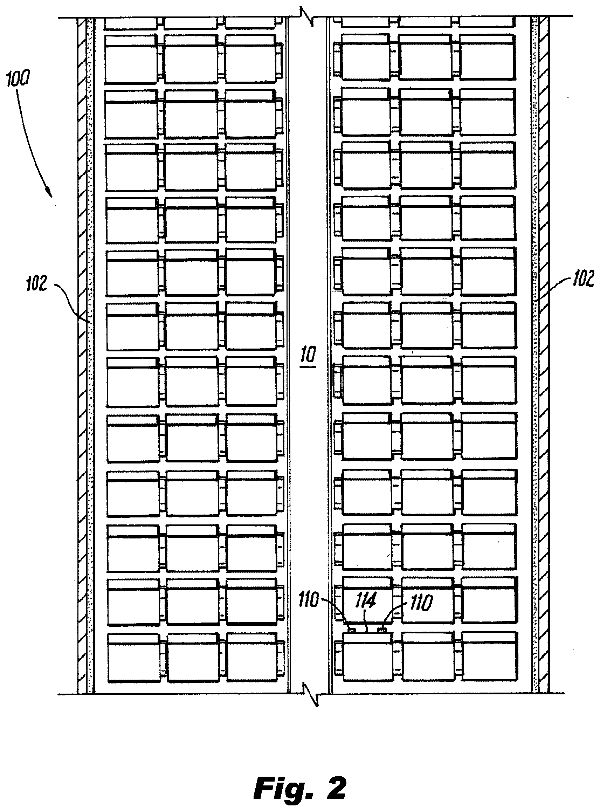

Reference will now be made to the drawings wherein like reference numerals identify similar structural features or aspects of the subject disclosure. For purposes of explanation and illustration, and not limitation, a schematic view of an exemplary embodiment of a system monitoring cabin air in accordance with the disclosure is shown in Fig. 1 and is designated generally by reference character 100. Other embodiments of systems in accordance with the disclosure, or aspects thereof, are provided in Fig. 2-5 as will be described. The systems and methods described herein can be used to collect a representative sample of pathogens within a pressurized cabin using collectors made from a collector material, e.g. filter material, adhesive tape, or the like. Embodiments of the present disclosure can be used in a variety of spaces, such as inside an aircraft cabin, train cabin, or the like, and allows for the detection of a virus or other contaminant.

As shown in Figs. 1-2, a system 100 for collecting a biological sample from a passenger cabin includes a collector 102, e.g. a continuous strip of collector material 102, positioned within the passenger cabin 10 in a cabin air outflow flow path 104. Cabin air outflow flow path 104 is generated by the pressurized air entering into cabin at inlets 109. As cabin air moves toward a cabin air outlet 107 it will impinge upon a surface of collector material 102 or run parallel to the surface of collector material 102, causing particulates, droplets or the like to collect on the collector material 102. Cabin air outlet 107 is defined between cabin floor 115 and hull wall 101 and goes down to a cargo area 5 and/or common ECS outlet. Collector 102 runs along the length of the cabin 10 located between the aircraft hull wall 101 and the passenger cabin 10. With continuous strip of collector material 102, only a small amount of pieces will need to be processed in order to extract a concentrated sample. In the embodiment of Fig. 1, system 100 also includes collectors 106, e.g. a group of individual pieces of collector material 106, on a cabin floor 115. The individual pieces of collector material 106 are positioned more proximate to a hull wall 101 than a cabin aisle 117.

With continued reference to Figs. 1-2, the embodiment of system 100 shown in Figs. 1 and 2 also includes collectors 108, e.g. a group of individual pieces of collector material 108, on an interior surface of a hull wall 101 and additional collectors 110, e.g. a group of individual pieces of collector material 110, on a passenger seat 116 back 114. Collectors 108 and 110 are positioned at a height *H* more proximate to a top 112 of seat 116 than a cabin floor 115.

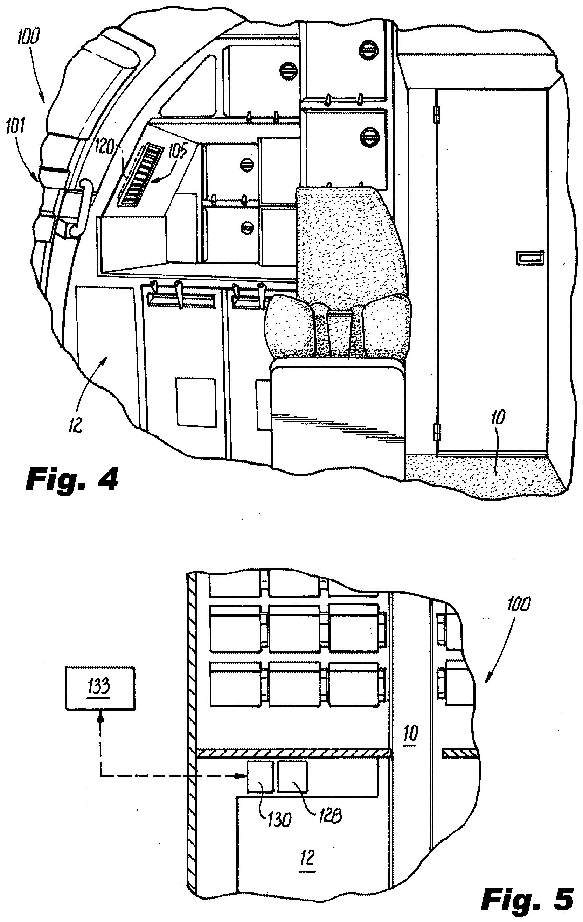

With reference now to Figs. 4-5, a collector 120, e.g. an individual piece of filter material 120, is positioned in a galley insert 12 air outflow flow path 105, such that airflow flowing out of galley insert 12 impinges on the filter material 120 and is filtered as it goes through filter material 120. The aircraft galley 12 includes a pathogen identifying testing unit 128, such as a Polymerase Chain Reaction (PCR) testing unit, within the aircraft galley insert 12. The pathogen identifying testing unit 128 can include a communication unit 130 for communicating results remotely, as shown schematically in Fig. 5 by the arrow in broken lines. The communication unit 130 can be configured to communicate the results to an external location, such as a central data center, or a destination airport, for example.

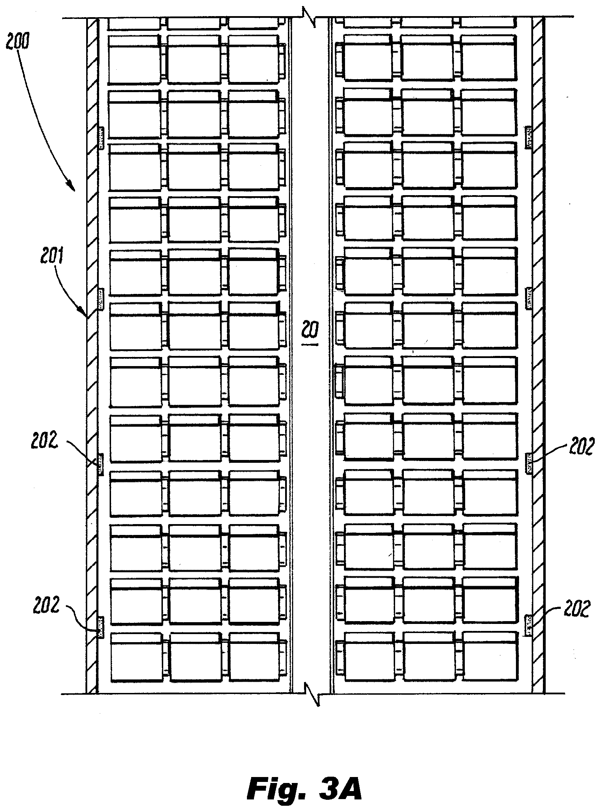

As shown in Figs. 3A-3B, another embodiment of a system 200 for collecting a biological sample from a passenger cabin 20. System 200 is the same as system 100 except that instead of collector 102 running as a continuous lengthwise strip, the collector 202 includes a series of individual pieces of collector material 202 spaced apart along lengthwise sides of the passenger cabin 20 positioned within a cabin air outflow flow path 204 between a hull wall 201 and the passenger cabin 20. Cabin air outflow flow path 204 is generated by the pressurized air entering into cabin at inlets 209. A cabin air outlet 207 is defined between cabin floor 215 and hull wall 201 opens down to a cargo area and/or common environmental control system (ECS) outlet. Each individual piece 202 of collector material is spaced apart by a given number of rows of seats. In accordance with the embodiment in Fig. 3A, each piece 202 is spaced apart by three rows of seats. Similar to system 100, the pieces 202 are located near the cabin floor 215 between the aircraft hull 201 and the passenger cabin 20. The pressurized cabin air flow flows in a similar manner to that of system 100. Because of the spacing in system 200, more collector material pieces 202 may need to be processed in order to extract a concentrated sample.

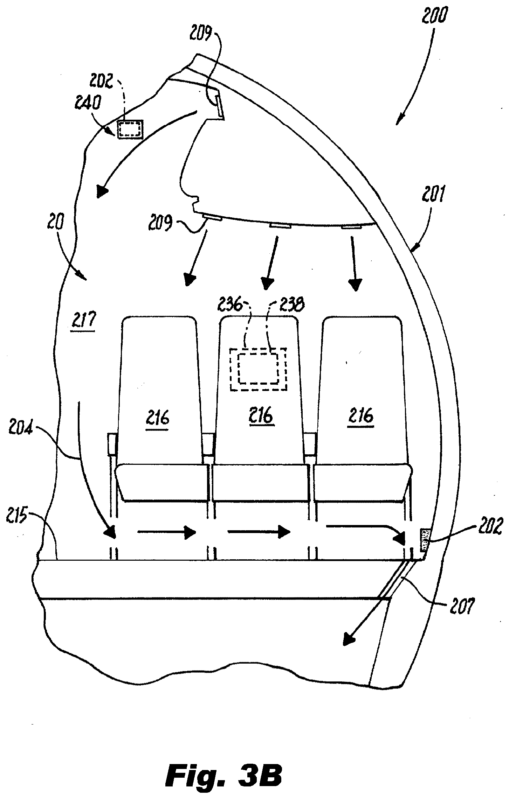

As shown in Fig. 3B, the outflow flow path 204 can be defined at least in part through the in-flight entertainment (IFE) system 236 having filter racks 238. In embodiments, the IFE can be located in a rear portion of a passenger seat (e.g. similar to that of collector 110) and can include a graphical user interface, such as a screen. The filter racks 238 can be located behind the screen to extract heat from the IFE hardware system. In certain embodiments, the filter already included in the filter rack 238 can be used or modified to be used as a collector material 202, and/or in certain embodiments, a separate or additional collector material 202 can be included in the rack in place of, or in addition to the existing filter.

Still with reference to Fig. 3B, the cabin air outflow flow path 204 can be a cabin temperature outflow flow path defined on or near a ceiling 240 of the cabin, for example near cabin inlets 209. Here, the cabin outflow path can include a grill having an existing filter medium therein, and the collector 202 can be or include an additional or alternative collector material 202 placed within the grill. In embodiments, the collector(s) as described can be located in any suitable outflow path, for example, in the ceiling 240 as shown, and additional collectors 202 can be included at the entrance halls, near or adjacent to the lavatories, near or adjacent the galleys, and/or any other suitable location in which may experience heavier passenger traffic.

A method for collecting particulates from cabin air includes capturing particulates in at least one of a passenger cabin, e.g. cabin 10 or 20, or a cabin air outflow flow path, e.g., cabin air outflow flow path 104 or 204, with a collector, e.g. collectors 102, 202, 106, 108, 110 or 120, for a period of time. The method includes removing the collector from at least one of the passenger cabin or air outflow flow path for testing. The method includes placing a clean collector into at least one of the passenger cabin or a cabin air outflow flow path for use during another period of time. The method includes conducting a pathogen identifying test such as Polymerase Chain Reaction (PCR) test, e.g. with pathogen identifying testing unit 128, on at least one particulate captured in the collector. The method includes relaying a result of the PCR test to a central data center, e.g. central data center 133, with a communication unit, e.g. communication unit 130. The central data center can be at a destination airport, governmental agency, or the like. The systems 100 and 200 are configured and adapted to collect representative sample/s of airborne pathogens within the pressurized cabin using collector pieces, e.g. filter material pieces.

The methods and systems of the present disclosure, as described above and shown in the drawings, provide for systems for monitoring aircraft air with superior properties including allowing for the detection of a virus or other contaminant. The systems and methods of the present invention can apply to aircraft travel, or the like. While the apparatus and methods of the subject disclosure have been shown and described with reference to preferred embodiments, those skilled in the art will readily appreciate that changes and/or modifications may be made thereto without departing from the scope of the claims.

## Claims

1. A system (100;200) for collecting a biological sample from a passenger cabin comprising:
a collector (102,106,108,110,120,202) for collecting particulate samples of the biological sample positioned within at least one of a passenger cabin (10) or a cabin air outflow flow path (104);
wherein the collector (102) includes a continuous strip of collector material positioned along a lengthwise side of the passenger cabin between a hull wall (101) and the passenger cabin.

2. The system as recited in claim 1, wherein the collector (202) includes a series of individual pieces of collector material spaced apart along a lengthwise side of the passenger cabin between a hull wall (201) and the passenger cabin (20); optionally wherein each individual piece of collector material is spaced apart by a given number of rows of seats.

3. The system as recited in claim 1, or 2, wherein the collector (106) includes a plurality of individual pieces of collector material below a row of seats; optionally wherein the plurality of individual pieces of collector material are more proximate to a hull wall than a cabin aisle.

4. The system as recited in any preceding claim, wherein the collector (108) includes a group of individual pieces of collector material on a hull wall (101) or a passenger seat back (114) at a height (H) more proximate to a top of a passenger seat than a cabin floor (115).

5. The system as recited in claim 1, wherein the collector (120) is positioned within the cabin air outflow flow path (105), wherein the cabin air outflow flow path is defined by a space between an aircraft hull and a cabin floor.

6. The system as recited in claim 1, wherein the collector is positioned within the cabin air outflow flow path, wherein the outflow flow path is defined at least in part through an in-flight entertainment system, IFE (236), filter racks (238).

7. The system as recited in any preceding claim, wherein the collector includes a plurality of individual pieces of filter material.

8. The system as recited in claim 1, wherein the collector is positioned within the cabin air outflow flow path, wherein the cabin air outflow flow path is a galley insert air outflow flow path (105), or wherein the cabin air outflow flow path is a cabin temperature outflow flow path.

9. The system of any preceding claim, wherein the particulate samples include droplets and/or gases exhaled from passengers throughout a duration of a flight.

10. The system as recited in any preceding claim, wherein the collector is configured and adapted to be removed from the passenger cabin or cabin air outflow flow path for testing.

11. The system of claim 1, wherein the cabin air outflow flow path is a general cabin outlet flow path and wherein the collector is positioned across the general cabin outlet flow path.

12. The system of any preceding claim, further comprising an aircraft galley proximate to the passenger cabin and a pathogen identifying testing unit within the aircraft galley; optionally wherein the pathogen identifying testing unit includes a communication unit (130) for communicating results remotely, and wherein the pathogen identifying testing unit is or includes a Polymerase Chain Reaction, PCR, testing unit (128).

13. A method for collecting particulates from cabin air comprising:
capturing particulates of a biological sample in a passenger cabin with a collector (102,106,108,110,120,202) for a period of time;
removing the collector from at least one of the passenger cabin or air outflow flow path for testing, wherein the collector (102) includes a continuous strip of collector material positioned along a lengthwise side of the passenger cabin between a hull wall (101) and the passenger cabin; and
placing a clean collector into at least one of the passenger cabin or a cabin air outflow flow path for use during another period of time.

14. The method of claim 13, further comprising conducting a pathogen identifying test on at least one particulate captured in the collector; and optionally further comprising relaying a result of the pathogen identifying test to a central data center (133).

## Patentansprüche

1. System (100; 200) zum Entnehmen einer biologischen Probe aus einer Passagierkabine, umfassend:
einen Entnehmer (102,106,108,110,120,202) zum Entnehmen von Partikelproben der biologischen Probe, der innerhalb mindestens einer von einer Passagierkabine (10) oder einem Kabinenluft-Abflussströmungsweg (104) positioniert ist;
wobei der Entnehmer (102) einen durchgehenden Streifen Entnehmermaterial beinhaltet, der entlang einer Längsseite der Passagierkabine zwischen einer Rumpfwand (101) und der Passagierkabine positioniert ist.

2. System nach Anspruch 1, wobei der Entnehmer (202) eine Reihe von einzelnen Stücken Entnehmermaterial beinhaltet, die entlang einer Längsseite der Passagierkabine zwischen einer Rumpfwand (201) und der Passagierkabine (20) beabstandet sind; wobei jedes einzelne Stück Entnehmermaterial optional um eine bestimmte Anzahl von Sitzreihen beabstandet ist.

3. System nach Anspruch 1 oder 2, wobei der Entnehmer (106) eine Vielzahl von einzelnen Stücken Entnehmermaterial unter einer Sitzreihe beinhaltet; wobei sich die Vielzahl von einzelnen Stücken Entnehmermaterial optional näher an einer Rumpfwand als an einem Kabinengang befindet.

4. System nach einem der vorhergehenden Ansprüche, wobei der Entnehmer (108) eine Gruppe von einzelnen Stücken Entnehmermaterial an einer Rumpfwand (101) oder einer Passagiersitz-Rückenlehne (114) in einer Höhe (H) beinhaltet, die sich näher an einer Oberseite eines Passagiersitzes als an einem Kabinenboden (115) befindet.

5. System nach Anspruch 1, wobei der Entnehmer (120) innerhalb des Kabinenluft-Abflussströmungswegs (105) positioniert ist, wobei der Kabinenluft-Abflussströmungsweg durch einen Raum zwischen einem Flugzeugrumpf und einem Kabinenboden definiert ist.

6. System nach Anspruch 1, wobei der Entnehmer innerhalb des Kabinenluft-Abflussströmungswegs positioniert ist, wobei der Abflussströmungsweg mindestens teilweise durch ein Bordunterhaltungssystem, IFE (236), Filtergestelle (238) definiert ist.

7. System nach einem der vorhergehenden Ansprüche, wobei der Entnehmer eine Vielzahl von einzelnen Stücken Filtermaterial beinhaltet.

8. System nach Anspruch 1, wobei der Entnehmer innerhalb des Kabinenluft-Abflussströmungswegs positioniert ist, wobei der Kabinenluft-Abflussströmungsweg ein Bordkücheneinsatz-Abflussströmungsweg ( 105 ) ist oder wobei der Kabinenluft-Abflussströmungsweg ein Kabinentemperatur-Abflussströmungsweg ist.

9. System nach einem der vorhergehenden Ansprüche, wobei die Partikelproben Tröpfchen und/oder Gase beinhalten, die von Passagieren während der Dauer eines Fluges ausgeatmet werden.

10. System nach einem der vorhergehenden Ansprüche, wobei der Entnehmer dazu konfiguriert und angepasst ist, zu Testzwecken aus der Passagierkabine oder dem Kabinenluft-Abflussströmungsweg entfernt zu werden.

11. System nach Anspruch 1, wobei der Kabinenluft-Abflussströmungsweg ein allgemeiner Kabinen-Abflussströmungsweg ist und wobei der Entnehmer quer über dem allgemeinen Kabinen-Abflussströmungsweg positioniert ist.

12. System nach einem der vorhergehenden Ansprüche, ferner umfassend eine Flugzeugbordküche in der Nähe der Passagierkabine und eine Testeinheit zum Identifizieren von Pathogenen innerhalb der Flugzeugbordküche; wobei die Testeinheit zum Identifizieren von Pathogenen optional eine Kommunikationseinheit (130) zum Fernkommunizieren von Ergebnissen beinhaltet und wobei die Testeinheit zum Identifizieren von Pathogenen eine Testeinheit für Polymerase-Kettenreaktions, PCR, (128) ist oder beinhaltet.

13. Verfahren zum Entnehmen von Partikeln aus der Kabinenluft, umfassend:
Einfangen von Partikeln einer biologischen Probe in einer Passagierkabine mit einem Entnehmer (102, 106, 108, 110, 120, 202) über einen bestimmten Zeitraum;
Entfernen des Entnehmers aus mindestens einem von der Passagierkabine oder einem Luft-Abflussströmungsweg zu Testzwecken, wobei der Entnehmer (102) einen durchgehenden Streifen Entnehmermaterial beinhaltet, der entlang einer Längsseite der Passagierkabine zwischen einer Rumpfwand (101) und der Passagierkabine positioniert ist; und
Platzieren eines sauberen Entnehmers in mindestens einer von der Passagierkabine oder einem Kabinenluft-Abflussströmungsweg zur Verwendung während eines anderen Zeitraums.

14. Verfahren nach Anspruch 13, ferner umfassend Durchführen eines Tests zum Identifizieren von Pathogenen mit mindestens einem in dem Entnehmer eingefangenen Partikel; und optional ferner umfassend Weiterleiten eines Ergebnisses des Tests zum Identifizieren von Pathogenen an ein zentrales Datenzentrum (133).

## Revendications

1. Système (100 ; 200) de collecte d'un échantillon biologique dans une cabine de passagers comprenant :
un collecteur (102,106,108,110,120,202) pour collecter des échantillons particulaires de l'échantillon biologique positionné dans au moins l'une d'une cabine de passagers (10) ou l'un d'un trajet d'écoulement de sortie d'air de cabine (104) ;
dans lequel le collecteur (102) inclut une bande continue de matériau collecteur positionnée le long d'un côté longitudinal de la cabine de passagers entre une paroi de coque (101) et la cabine de passagers.

2. Système selon la revendication 1, dans lequel le collecteur (202) inclut une série de pièces individuelles de matériau collecteur espacées le long d'un côté longitudinal de la cabine de passagers entre une paroi de coque (201) et la cabine de passagers (20) ; éventuellement, dans lequel chaque pièce individuelle de matériau collecteur est espacée d'un nombre donné de rangées de sièges.

3. Système selon la revendication 1 ou 2, dans lequel le collecteur (106) inclut une pluralité de pièces individuelles de matériau collecteur sous une rangée de sièges ; éventuellement dans lequel la pluralité de pièces individuelles de matériau collecteur est plus proche d'une paroi de coque que d'un couloir de cabine.

4. Système selon une quelconque revendication précédente, dans lequel le collecteur (108) inclut un groupe de pièces individuelles de matériau collecteur sur une paroi de coque (101) ou un dossier de siège de passager (114) à une hauteur (H) plus proche du sommet d'un siège de passager que d'un plancher de cabine (115).

5. Système selon la revendication 1, dans lequel le collecteur (120) est positionné à l'intérieur du trajet d'écoulement de sortie d'air de cabine (105), dans lequel le trajet d'écoulement de sortie d'air de cabine est défini par un espace entre une coque d'aéronef et un plancher de cabine.

6. Système selon la revendication 1, dans lequel le collecteur est positionné à l'intérieur du trajet d'écoulement de sortie d'air de cabine, dans lequel le trajet d'écoulement de sortie est défini au moins en partie par un système de divertissement en vol, IFE (236), des supports de filtre (238).

7. Système selon une quelconque revendication précédente, dans lequel le collecteur inclut une pluralité de pièces individuelles de matériau filtrant.

8. Système selon la revendication 1, dans lequel le collecteur est positionné à l'intérieur du trajet d'écoulement de sortie d'air de cabine, dans lequel le trajet d'écoulement de sortie d'air de cabine est un trajet d'écoulement de sortie d'air d'insert d'office (105), ou dans lequel le trajet d'écoulement de sortie d'air de cabine est un trajet d'écoulement de sortie de température de cabine.

9. Système selon une quelconque revendication précédente, dans lequel les échantillons particulaires incluent des gouttelettes et/ou des gaz exhalés par les passagers pendant la durée d'un vol.

10. Système selon une quelconque revendications précédentes, dans lequel le collecteur est configuré et adapté pour être retiré de la cabine de passagers ou du trajet d'écoulement de sortie d'air de cabine à des fins de test.

11. Système selon la revendication 1, dans lequel le trajet d'écoulement de sortie d'air de cabine est un trajet d'écoulement de sortie de cabine général et dans lequel le collecteur est positionné en travers du trajet d'écoulement de sortie de cabine général.

12. Système selon une quelconque revendication précédente, comprenant également un office d'aéronef à proximité de la cabine de passagers et une unité de test d'identification d'agent pathogène à l'intérieur de l'office d'aéronef ; éventuellement dans lequel l'unité de test d'identification d'agent pathogène inclut une unité de communication (130) pour communiquer les résultats à distance, et dans lequel l'unité de test d'identification d'agent pathogène est ou inclut une unité de test de réaction en chaîne par polymérase, PCR (128).

13. Procédé de collecte de particules dans l'air d'une cabine comprenant :
la capture de particules d'un échantillon biologique dans une cabine de passagers avec un collecteur (102, 106, 108, 110, 120, 202) pendant une période de temps ;
le retrait du collecteur d'au moins une de la cabine de passagers ou d'un du trajet de sortie d'air aux fins de test, dans lequel le collecteur (102) inclut une bande continue de matériau collecteur positionnée le long d'un côté longitudinal de la cabine de passagers entre une paroi de coque (101) et la cabine de passagers ; et
le placement d'un collecteur propre dans au moins une de la cabine de passagers ou un du trajet d'écoulement d'air de cabine de passagers pour l'utiliser pendant une autre période de temps.

14. Procédé selon la revendication 13, comprenant également la réalisation d'un test d'identification d'agent pathogène sur au moins une particule capturée dans le collecteur ; et comprenant également facultativement la transmission d'un résultat du test d'identification d'agent pathogène à un centre de données central (133).
